# EUROPEAN PATENT SPECIFICATION

(11) **EP 1 536 777 B1**
(45) Date of publication and mention of the grant of the patent: **14.02.2007**
(21) Application number: 03753579.6
(22) Date of filing: 12.08.2003
(51) Int. Cl.: A61K 31/00, A61K 31/4985, A61K 31/277, A61K 31/352, A61K 31/223, A61K 31/415, A61K 31/325, A61K 45/06, A61P 31/04, A61P 15/14

(54) **USE OF NF-KAPPA B INHIBITORS FOR THE TREATMENT OF MASTITIS**
VERWENDUNG VON NF-KAPPA B HEMMERN ZUR BEHANDLUNG VON BRUSTDRÜSENENTZÜNDUNG
UTILISATION D'INHIBITEURS DU NF-KAPPA B POUR LE TRAITEMENT DE LA MASTITE

(30) Priority: 14.08.2002 EP 02078352
(43) Date of publication of application: 08.06.2005
(73) Proprietor: JANSSEN PHARMACEUTICA N.V., 2340 Beerse (BE)
(72) Inventor: Boulanger, Delphine, 4000 Liège (BE); Bureau, Fabrice, 4000 Liège (BE); Lekeux, Pierre, 4000 Liège (BE)
(74) Representative: Verberckmoes, Filip Gerard
(86) International application number: PCT/EP2003/050373
(87) International publication number: WO 2004/016253

(56) References cited:
- WO-A-00/56341
- WO-A-01/21202
- WO-A-96/13266
- BOUCHARD L ET AL: "Nitric oxide production during endotoxin-induced mastitis in th cow" JOURNAL OF DAIRY SCIENCE, (DEC 1999) VOL. 82, NO. 12, PP. 2574-2581. PUBLISHER: AMER DAIRY SCIENCE ASSOC, 1111 N DUNLAP AVE, SAVOY, IL 61874. ISSN: 0022-0302., XP001109451 AGR & AGRI FOOD CANADA, DAIRY & SWINE RES & DEV CTR, POB 90, 2000 ROUTE 108 E, LENNOXVILLE, PQ J1M 1Z3, CANADA (Reprint);AGR & AGRI FOOD CANADA, DAIRY & SWINE RES & DEV CTR, LENNOXVILLE, PQ J1M 1Z3, CANADA; MCGILL UNIV DEPT ANIM SCI, ST ANNE BELLEVUE, PQ H9X 3V9, CANADA
- DATABASE BIOSIS [Online] BIOSCIENCES INFORMATION SERVICE, PHILADELPHIA, PA, US; May 2001 (2001-05) ZOUKI CHRISTINE ET AL: "Peroxynitrite mediates cytokine-induced IL-8 gene expression and production by human leukocytes." Database accession no. PREV200100325362 XP002226807 & JOURNAL OF LEUKOCYTE BIOLOGY, vol. 69, no. 5, May 2001 (2001-05), pages 815-824, ISSN: 0741-5400
- ONODA M (REPRINT) ET AL: "Effect of curcumin on the production of nitric oxide by cultured rat mammary gland" NITRIC OXIDE-BIOLOGY AND CHEMISTRY, (OCT 2000) VOL. 4, NO. 5, PP. 505-515 PUBLISHER: ACADEMIC PRESS INC, 525 B ST, STE 1900, SAN DIEGO, CA 92101-4495. ISSN: 1089-8603., XP001133827 NATL INST RADIOL SCI, RES GRP 1, INAGE KU, 4-9-1 ANAGAWA, CHIBA 2638555, JAPAN (Reprint)
- DATABASE MEDLINE [Online] 15 December 1968 (1968-12-15) NEWBOULD F H: "Epizootiology of mastitis due to staphylococcus aureus." Database accession no. NLM5749697 XP002226808 & JOURNAL OF THE AMERICAN VETERINARY MEDICAL ASSOCIATION. UNITED STATES 15 DEC 1968, vol. 153, no. 12, 15 December 1968 (1968-12-15), pages 1683-1687, ISSN: 0003-1488

## Description

The present invention relates to the use of NF-κB (NF-kappa B) inhibitors according to claim 1 for the manufacture of a medicament for the treatment of mastitis.

Mastitis is one of the most costly diseases in dairy herds. Costs mainly arise from decreased milk production and quality, therapeutic interventions, loss of antibiotic-contaminated milk and extra labor. Mastitis, defined as an inflammatory condition of the mammary gland, is usually caused by pathogenic bacteria. The most common pathogens that provoke mastitis include *Staphylococcus aureus, Streptococcus agalactiae, Streptococcus dysgalactiae, Streptococcus uberis, Arcanobacterium pyogenes* and coliforms. These microorganisms can invade the udder through the teat canal and produce inflammation of the milk-producing tissue causing the formation of scar tissue which, once formed, may cause a permanent reduction in the cow's milk production. An infection can also alter the composition, quantity, appearance and quality of the milk.

Mastitis in dairy cattle is usually treated with antibiotics. However it has been found to be desirable to replace such treatment with antibiotics with treatment by non-antibiotic chemo-therapeutic drugs to avoid building up of resistance of bacterial strains.
Furthermore, milk produced by dairy cattle on an antibiotic treatment has to be discarded as it contains residual antibiotics.

NF-κB (nuclear factor-kappa B) is a collective name for inducible dimeric transcription factors composed of members of the Rel family of DNA-binding proteins that recognize a common sequence motif. NF-κB is found in essentially all cell types and is involved in activation of an exceptionally large number of genes in response to infections, inflammation, and other stressful situations requiring rapid reprogramming of gene expression.

Bouchard *et al.* disclose in J. Diary Science, 1999, 82(12), 2574-2581 that the iNOS inhibitor aminoguanidine can prevent NO release during mastitis and suggest a potential therapeutic value. It is further mentioned that it would be interesting to investigate the anti-inflammatory potential of substances which interfere with the activation of NF-κB in a model of mastitis.

WO-A-0056341 discloses the use of a NF-κB inhibitor for the treatment of bacterial-mediated disorders due to Gram negative or Gram positive bacteria.

Surprisingly, it has been found that compounds capable of inhibiting the activation of NF-κB, *i.e.* NF-κB inhibitors according to claim 1, can be of use in the treatment of mastitis.

Known NF-κB inhibitors are e.g. :
- 15d-PGJ2 (15-deoxy-Δ.12,14-prostaglandin J2) (see *Proc. Natl. Acad. Sci. USA,* 97(9), 4844-4849 (2000));
- tepoxalin (see *Immunology Letters,* 53(2,3), 109-113 (1996));
- cycloepoxydon and its (+)- and (-)- stereoisomers (see *J. Am. Chem. Soc.,* 123(45), 11308-11309 (2001));
- sodium diethyldithiocarbamate (Na-DDTC) (see *Brain Research Bulletin,* 55/3, 375-386 (2001));
- gliotoxin, MG-132 (N-[(phenylmethoxy)carbonyl]-L-leucyl-N-[(1S)-1-formyl-3-methylbutyl]-L-Leucinamide), BAY 11-7082 (3-[[4-(1,1-dimethylethyl)phenyl]-sulfonyl]-2-propenenitrile), BAY 11-7085 ((E)-(3-[[4-(1,1-dimethylethyl)phenyl]-sulfonyl]-2-propenenitrile), (see *Human Pathology,* 31 (12), 1482-1490 (2000));
- cyclopentenone prostaglandin A1 (PGA1), (see *Proc. Natl. Acad. Sci. USA,* 94, 746-750 (1997));
- cyclopentabenzofurans as described in WO-00/08007; and
- [(1R)-3-methyl-1-[[(2S)-1-oxo-3-phenyl-2-[(pyrazinylcarbonyl)amino]propyl]-amino]butyl]-boronic acid also known under the INN name of bortezomib.

Other NF-κB inhibitors can be learned from literature and compounds can be tested for NF-κB inhibiting activity using assays known in the art. See for instance *Proc. Natl. Acad Sci. USA,* 94, 746-750 (1997).

The NF-κB inhibitors according to claim 1 for use in the treatment of mastitis can be specific inhibitors of NF-κB or can be non-specific.

The present invention provides the use of a NF-κB inhibitor according to claim 1 for the manufacture of a medicament for the treatment of mastitis.

As used herein, the term "subject" embraces warm-blooded animal species such as e.g. ruminants such as cattle, sheep, goats, buffalo etc.

There are a variety of forms or types of bovine mastitis, with varying severity and symptomatology, including the following :
- acute mastitis which is an abrupt and short-lived inflammation of the udder,
- chronic mastitis which is a moderate and persistent inflammation of the mammary gland.

Acute mastitis is generally a clinical form whereas chronic mastitis is often a subclinical form of the disease. Subclinical mastitis is a form of mastitis in which there is no swelling of the gland or observable abnormality of the milk, although there are changes in the milk, such as an increase in somatic cell count (SCC), that can be detected by special tests. This type of mastitis is by far the most prevalent and causes the greatest overall loss in most herds. It often is referred to as "hidden" mastitis. Clinical mastitis is a form of mastitis in which the abnormal conditions of the udder and secretion are observable. Mild clinical mastitis involves changes in the milk such as flakes, clots, and a watery or unusual appearance. Heat and sensitiveness of the udder are slight or absent, but there may be signs of swelling. Severe clinical mastitis involves a sudden onset with swelling of the infected quarter which is hot, hard and sensitive. The milk appears abnormal and milk production drops. Sometimes, in addition to the local effects in the udder, the cow herself becomes sick. There are signs of fever, rapid pulse, depression, weakness and loss of appetite. The combination of these conditions often is referred to as acute systemic mastitis, because not only the udder, but the whole animal is affected.

The effective amount of NF-κB inhibitor according to claim 1 for the treatment of mastitis will be determined using routine optimization techniques and are dependent upon the particular condition to be treated, the condition of the subject, the route of administration, the formulation, and the judgment of the practitioner and other factors evident to those skilled in the art. An effective amount may be achieved by multiple dosing.

Compositions comprising a NF-κB inhibitor according to claim 1 may be administered intravascularly, intraperitoneally, subcutaneously, intramuscularly, or by intramammary infusion using forms known in the pharmaceutical art. For intravascular, intraperitoneal, subcutaneous, intramuscular or intramammary administration, active drug components may be combined with a suitable carrier such as water, saline, aqueous dextrose, and the like. Veterinary formulations comprising a NF-κB inhibitor are usually prepared by mixing said NF-κB inhibitor with an excipient, diluting by an excipient or enclosing within a carrier that can be in the form of a capsule, sachet, or other container. When the excipient serves as a diluent, it can be a solid, semi-solid, or liquid material that acts as a vehicle, carrier or medium for the NF-κB inhibitor. Examples of suitable excipients include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, syrup, and methyl cellulose. The formulations can additionally include: lubricating agents such as talc, magnesium stearate, and mineral oil; wetting agents; emulsifying and suspending agents; preserving agents such as methyl- and propylhydroxybenzoates; sweetening agents; and flavoring agents. The compositions of the invention can be formulated to provide quick, sustained or delayed release of the active ingredient after administration to the host by procedures known in the art.

Intramammary infusions may be oil based, e.g. a vegetable oil such as peanut oil or a non-vegetable oil such as mineral oil and may further include a thickening agent and optionally also a surfactant.

Compositions can be in the form of tablets, pills, powders, lozenges, sachets, cachets, elixirs, suspensions, emulsions, solutions, infusions, syrups, aerosols, ointments, soft and hard gelatin capsules, suppositories, sterile injectable solutions and sterile packages powders. Regardless of the route of administration selected, these compositions are formulated into pharmaceutically acceptable dosage forms by conventional methods known to those skilled in the art.

The NF-κB inhibitors according to claim 1 may also be used in combination therapy with antibiotics selected from beta-lactam antibiotics selected from ampicillin, cloxacillin, hetacillin, nafcillin, penicillin G, procaine penicillin; cephalosporins selected from cefoperazone, cefuroxime, cefalonium, cefapirin, cefoxazole, cefracetrile; aminoglycoside antibiotics selected from framycetin, neomycin, novobiocin, streptomycin; macrolide antibiotics selected from erythromycin; tetracyclines selected from chlortetracycline, oxytetracycline; or polypeptide antibiotics selected from polymyxin B.

### Experimental details

The level of NF-κB activity in milk cells from healthy and acute and chronic mastitis-affected cows was measured.

### A. Measurement of NF-KB activity in mastitis affected cows

### 1. Animals

Twenty healthy, six acute and twenty chronic mastitis-affected Holstein-Friesian cows were used in this study. Healthy cows had low somatic cell count (SCC) (<10⁵ cells/ml) and were free of any clinical sign of mastitis. Acute mastitis-affected cows had high SCC (> 4x10⁵ cells/ml) and exhibited clinical signs of illness. Neither healthy nor acute mastitis-affected cows had earlier record of udder disease, as measured by clinical symptoms and monthly SCC recordings. Chronic mastitis-affected cows had persistently increased SCC (> 4 x 10⁵ cells/ml), as determined by monthly SCC measurements, but were devoid of any clinical sign of the disease. All the cows were between 2 and 7 years old, had a lactation number ranging from 1 to 5 and were lactating for 1 to 6 months. None of the cows received any treatment during the month preceding the experiments.

### 2. Sterile Milk Sampling

Milk was collected using a sterile teat cannula infusion apparatus. This apparatus consisted of a sterile and pyrogen-free cannula inserted into the teat canal and connected to the free end of an infusion set attached to a 2000 ml sterile collection bag. Milk samples were taken from one quarter. Before sampling, the quarter was thoroughly disinfected with a solution containing 70% alcohol and 30% chlorohexidine. Immediately after milk sampling, an aliquot of 50 µl was the subject of a microbiological analysis. Samples that were found to be contaminated by bacteria were excluded from the study.

### 3. Somatic Cell Count

Immediately after milk collection, 50 ml of each milk sample was shipped to a specialized laboratory (Laboratory of the Milk Committee, Battice, Belgium), where a somatic cell count was performed following classical procedure.

### 4. Isolation of Milk Cells

Milk samples were first centrifuged (300 g) for 30 minutes at 4°C. Pellets were then washed in phosphate buffered saline (PBS) and centrifuged (300 g) for another 30 minutes at 4°C. The pelleted cells were finally resuspended in 1 ml of PBS before protein extraction. Cell differentials were performed on cytospin preparations stained with Diff-Quick (Dade Behring, Dudingen, Germany).

### 5. Protein Extraction

Isolated milk cells were centrifuged for 10 minutes at 300 g and the pellet was resuspended in 600 µl lysis buffer (0.1 % nonidet P-40; 10 mM Tris HC1, pH 7.4; 10 mM NaCl; 3 mM MgCl₂; 1 mM EDTA; 2 mM orthovanadate; 1 mM diisopropyl fluorophosphates (DFP); 10 µg/ml leupeptin; 10 µg/ml aprotinin). The cells were vortexed 15 seconds, kept on ice for 5 minutes, and centrifuged for 10 minutes at 300 g. The resulting pellet was resuspended in a KCl buffer (10 mM Hepes, pH 7.4; 400 mM KCI; 10% Glycerol; 2 mM EDTA; 1 mM Ethylene glycol-bis[β-aminoethyl ether]-N, N, N'N'-tetraacetic acid (EGTA); 1% nonidet P-40, 1 mM dithiothreitol (DTT); 2 mM orthovanadate; 10 µg/ml leupeptin; 10 µg/ml aprotinin; 1 mM DFP) and kept at 4°C for 10 minutes before centrifugation for 15 minutes at 12,000 g. The supernatant was diluted three times and stored at -80°C until use.

### 6. NF-κB Electrophoretic Mobility Shift Assays (EMSAs)

Binding reactions were performed for 30 minutes at room temperature with 5 µg of total protein extracts in 20 mM Hepes (pH 7.9), 10 mM KC1, 0.2 mM EDTA, 20% (v/v) glycerol, 1 % (w/v) acetylated bovine serum albumin, 3 µg of poly(dI-dC) (Amersham Pharmacia Biotech, Aylesbury, U.K.), 1 mM DTT (dithiothreitol), 1 mM phenylmethylsulfonyl fluoride, and 100,000 cpm of ³²P-labeled double-stranded oligonucleotide probes. Probes were prepared by annealing the appropriate single-stranded oligonucleotides (Eurogentech, Liège, Belgium) at 65°C for 10 minutes in 10 mM Tris, 1 mM EDTA, and 10 mM NaCl, followed by slow cooling to room temperature. The probes were labeled by end-filling with the Klenow fragment of *E. coli* DNA polymerase I (Roche, Mannheim, Germany), with [³²P]dATP and [³²P]dCTP (Dupont-New England Nuclear, Les Ulis, Fance). Labeled probes were purified by spin chromatography on Sephadex G-25 columns (Roche). DNA-protein complexes were separated from unbound probe on 4% native polyacrylamide gels at 150 V in 0.25 M Tris, 0.25 M sodium borate, and 0.5 mM EDTA, pH 8.0. Gels were vacuum-dried and exposed to x-ray film at -80°C for 12 hours. The amount of specific complexes was determined by photodensitometry of the autoradiography. To confirm specificity, competition assays were performed with a 50-fold excess of unlabeled wild-type and mutated probes. For supershift experiments, 1.5 µl of each antibody was incubated with the extracts 30 minutes before addition of the radiolabeled probe. The sequences of the oligonucleotides used in this work were as follows : wild-type palindromic κB probe, 5'-TTG GCA ACG GCA GGG GAA TTC CCC TCT CCT TAG GTT-3'; mutated palindromic κB probe, 5'-TTG GCA ACG GCA GAT CTA TTC CCC TCT CCT TAG GTT-3'.
The NF-κB activity was assessed by photodensitometry of the specific bands.

### 7. Results

Active NF-κB complexes were undetectable in milk cells from healthy cows, as determined by EMSAs. Conversely, protein extracts prepared from milk samples of acute mastitis-affected cows demonstrated a high NF-κB activity. In milk cells from cows suffering from chronic mastitis, NF-κB activity varied from low to high.

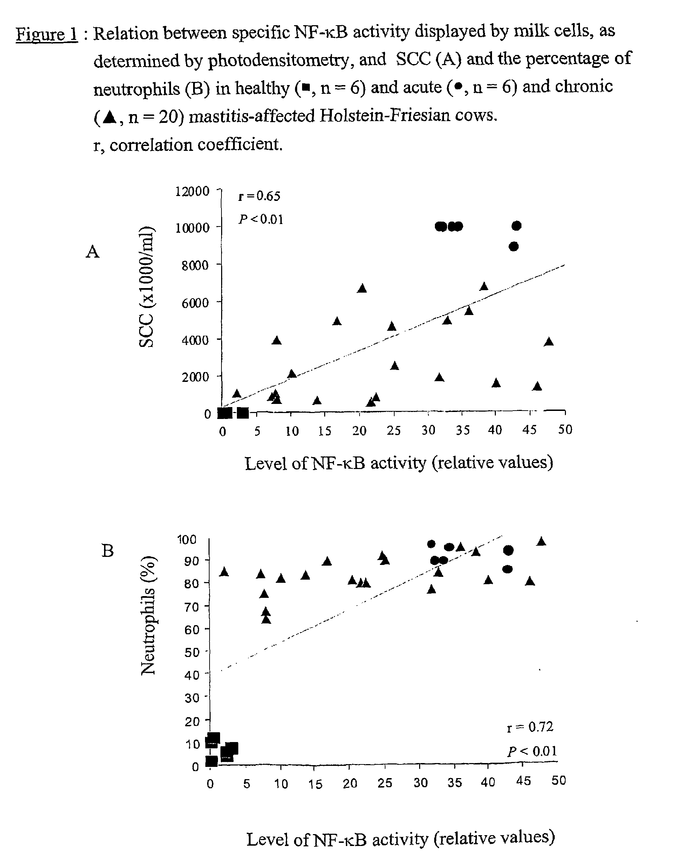

Linear regressions were carried out to assess the relation between the level of NF-κB activity in milk cells, as assessed by photodensitometry, and the degree of inflammation in the mammary gland. This regression analysis demonstrated a highly significant (*P* < 0.01) correlation between NF-κB activity and two markers of udder inflammation, namely SCC (Figure 1A) and the percentage of neutrophils in milk samples (Figure 1 B).

This study demonstrated that NF-κB activity was increased in milk cells from mastitis-affected cows and therefore NF-κB inhibitors are believed to be of potential use in the treatment of mastitis.

### B. Effects of administration of an NF-κB inhibitor on milk cells obtained from chronic mastitis-affected cows

### 1. Animals

Four chronic mastitis-affected Holstein-Friesian cows were used in this study. These cows had persistently increased SCC (> 4 x 10⁵ cells/ml), as determined by monthly SCC measurements, but were devoid of any clinical sign of the disease. None of the cows received any treatment during the month preceding the experiments.

### 2. Sterile Milk Sampling

Milk was collected as described above.

### 3. Somatic Cell Count

Immediately after milk collection, 50 ml of each milk sample was shipped to a specialized laboratory (Laboratory of the Milk Committee, Battice, Belgium), where a somatic cell count was performed following classical procedure.

### 4. Isolation of Milk Cells

Milk samples were first centrifuged (300 g) for 30 minutes at 4°C. Pellets were then washed in phosphate buffered saline (PBS) and centrifuged (300 g) for another 30 minutes at 4°C. The pelleted cells were finally resuspended in 1 ml of PBS before protein extraction. Cell differentials were performed on cytospin preparations stained with Diff-Quick (Dade Behring, Dudingen, Germany).

### 5. Culture and treatment of milk cells

Milk cells were cultured at 4 x 10⁶ per ml in RPMI 1640 medium (Life Technologies, Merelbeke, Belgium) supplemented with 1% glutamine, 10% foetal bovine serum, streptomycine 50 µg/ml, and penicillin 50 UI/ml. The cells were first incubated at 37°C in a 5% CO2-95% air mixture for 90 minutes then treated with various doses of NF-KB inhibitors (15-dPGJ2 at concentrations of 40 µM, 50 µM, 60 µM and 80 µM; or gliotoxine at a concentration of 1 µl/ml). Treated cells were incubated 3 hours before protein extraction.

### 6. Protein Extraction

See procedure described above.

### 7. NF-κB Electrophoretic Mobility Shift Assays (EMSAs)

See procedure described above for assessing NF-κB activity by photodensitometry.

### 8. Results

In untreated milk cells from chronic mastitis-affected cows, an elevated level of NF-κB activity was detected, as determined by EMSAs. Conversely, protein extracts prepared from milk cells treated with 15d-PGJ2 or glyotoxine demonstrated a reduced NF-κB activity.

### Description of the drawings

Figure 1 shows the relation between specific NF-κB activity displayed by milk cells, as determined by photodensitometry, and SCC (A) and the percentage of neutrophils (B) in healthy (■, n = 6) and acute (**•,** n = 6) and chronic (▲, n = 20) mastitis-affected Holstein-Friesian cows. r, correlation coefficient.

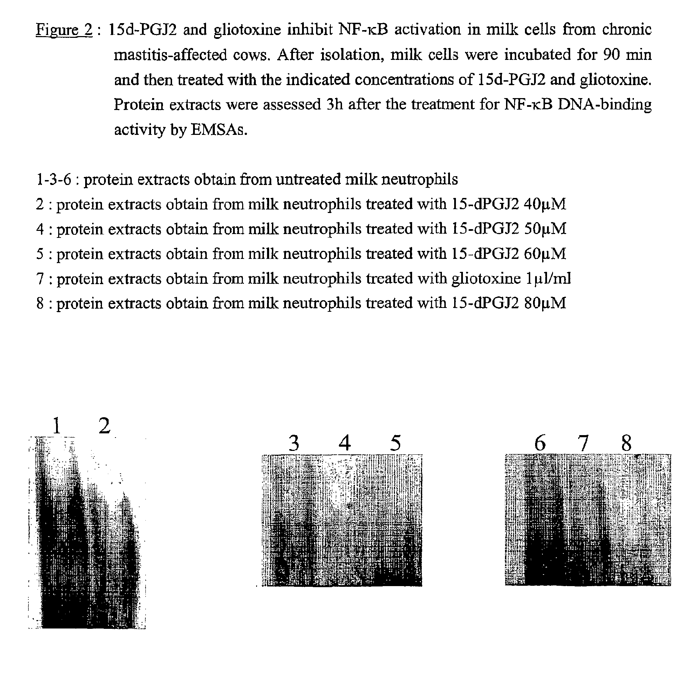

Figure 2 : 15d-PGJ2 and gliotoxine inhibit NF-κB activation in milk cells from chronic mastitis-affected cows. After isolation, milk cells were incubated for 90 min and then treated with the indicated concentrations of 15d-PGJ2 and gliotoxine. Protein extracts were assessed 3h after the treatment for NF-κB DNA-binding activity by EMSAs.

## Claims

1. Use of a NF-κB inhibitor selected from 15d-PGJ2, tepoxalin, cycloepoxydon, (+)-cycloepoxydon, (-)-cycloepoxydon, sodium diethyldithiocarbamate, gliotoxin, MG-132 (N- [(phenylmethoxy)carbonyl]-L-leucyl-N-[(1S)- formyl-3-methylbutyl]-L- Leucinamide), BAY 11-7082, (3-[[4-(1,1-dimethylethyl)phenyl]-sulfonyl]-2-propenenitrile), BAY 11-7085 ((E)-(3-[[4-(1,1-dimethylethyl)phenyl]-sulfonyl]-2-propenenitrile) or bortezomib for the manufacture of a medicament for the treatment of mastitis.

2. Use according to claim 1 wherein mastitis is chronic mastitis.

3. Use according to claim 1 wherein mastitis is acute mastitis.

4. Use according to any one of claims 1 to 3 in combination with an antibiotic selected from ampicillin, cloxacillin, hetacillin, nafcillin, penicillin G, procaine penicillin, cefoperazone, cefuroxime, cefalonium, cefapirin, cefoxazole, cefracetrile, framycetin, neomycin, novobiocin, streptomycin, erythromycin, chlortetracycline, oxytetracycline and polymyxin B.

5. Use according to any one of the preceding claims wherein the NF-κB inhibitor is to be administered intramammary.

## Patentansprüche

1. Verwendung eines NF-κB-Inhibitors, ausgewählt aus 15d-PGJ2, Tepoxalin, Cycloepoxydon, (+)-Cycloepoxydon, (-)-Cycloepoxydon, Natriumdiethyldithiocarbamat, Gliotoxin, MG-132 (N-[(Phenylmethoxy)carbonyl]-L-leucyl-N-[(15)-formyl-3-methylbutyl]-L-leucinamid), BAY 11-7082 (3-[[4-(1,1-Dimethylethyl)phenyl]-sulfonyl]-2-propennitril), BAY 11-7085 ((E)-(3-[[4-(1,1-Dimethylethyl)phenyl]-sulfonyl]-2-propennitril) oder Bortezomib zur Herstellung eines Medikamentes für die Behandlung von Mastitis.

2. Verwendung nach Anspruch 1, wobei die Mastitis chronische Mastitis ist.

3. Verwendung nach Anspruch 1, wobei die Mastitis akute Mastitis ist.

4. Verwendung nach einem der Ansprüche 1 bis 3 in Kombination mit einem Antibiotikum, ausgewählt aus Ampicillin, Cloxacillin, Hetacillin, Nafcillin, Penicillin G, Procain, Penicillin, Cefoperazon, Cefuroxim, Cefalonium, Cefapirin, Cefoxazol, Cefacetril, Framycetin, Neomycin, Novobiocin, Streptomycin, Erythromycin, Chlortetracyclin, Oxytetracyclin und Polymyxin B.

5. Verwendung nach einem der vorhergehenden Ansprüche, wobei der NF-κB-Inhibitor intramammär verabreicht werden soll.

## Revendications

1. Utilisation d'un inhibiteur de NF-κB choisi parmi la 15d-PGJ2, la tépoxaline, le cycloépoxydon, le (+)-cycloépoxydon, le (-)-cycloépoxydon, le diéthyldithiocarbamate de sodium, la gliotoxine, le MG-132 (N-[(phénylméthoxy) carbonyl]-L-leucyl-N-[(1S)-formyl-3-méthylbutyl]-L-leucinamide), le BAY 11-7082 (3-[[4-(1,1-diméthyléthyl)phényl]-sulfonyl]-2-propènenitrile), le BAY 11-7085 ((E)-(3-[[4-(1,1-diméthyléthyl)phényl]-sulfonyl]-2-propènenitrile) ou le bortézomib pour la fabrication d'un médicament destiné au traitement de la mastite.

2. Utilisation selon la revendication 1, **caractérisée en ce que** la mastite est la mastite chronique.

3. Utilisation selon la revendication 1, **caractérisée en ce que** la mastite est la mastite aiguë.

4. Utilisation selon l'une quelconque des revendications 1 à 3, en combinaison avec un antibiotique choisi parmi l'ampicilline, la cloxacilline, l'hétacilline, la nafcilline, la pénicilline G, la procaïne pénicilline, la céfopérazone, le céfuroxime, le céfalonium, la céfapirine, le céfoxazole, le céfacétrile, la framycétine, la néomycine, la novobiocine, la streptomycine, l'érythromycine, la chlortétracycline, l'oxytétracycline et la polymyxine B.

5. Utilisation selon l'une quelconque des revendications précédentes, **caractérisée en ce que** l'inhibiteur de NF-κB est destiné à une administration par voie intramammaire.
